(19) Europäisches Patentamt
European Patent Office
Office européen des brevets

(11) Veröffentlichungsnummer: **0 014 181**
**B1**

(12) # EUROPÄISCHE PATENTSCHRIFT

(45) Veröffentlichungstag der Patentschrift: **10.11.82**

(21) Anmeldenummer: **80810016.8**

(22) Anmeldetag: **21.01.80**

(51) Int. Cl.³: **C 12 P 17/18**, C 07 D 498/08, C 07 D 498/18, A 61 K 31/395 // (C07D498/18, 311/00, 307/00, 267/00),(C07D498/00, 307/00, 267/00),(C12P17/18, C12R1/365)

(54) **Neue antibiotisch wirksame Verbindungen, ihre fermentative Herstellung und sie enthaltende pharmazeutische Präparate.**

(30) Priorität: **25.01.79 CH 750/79**
**21.06.79 CH 5804/79**

(43) Veröffentlichungstag der Anmeldung: **06.08.80 Patentblatt 80/16**

(45) Bekanntmachung des Hinweises auf die Patenterteilung: **10.11.82 Patentblatt 82/45**

(84) Benannte Vertragsstaaten: **AT BE CH DE FR GB IT LU NL SE**

(56) Entgegenhaltungen:
**CH-A- 605 974**
**US-A-4 042 683**
**CHEMICAL ABSTRACTS, Band 77, Nr. 21, 20. November 1972, Seite 10, Nr, 135025x Columbus, Ohio, USA S.S. YANG et al.: «Rifamycin antibiotics, inhibitors of Rauscher murine leukemia virus reverse transcriptase and of purified DNA polymerases from human normal and leukemic lymphoblasts»**
**THE JOURNAL OF ANTIBIOTICS; Band XXIV, Nr. 4, 1976, Seiten 466–468 Ausg.: Japanese Antibiot. Research Association G. LANCINI et al.: «Rifamycin G, A further Product of Nocardia Mediterranei Metabolism»**

(73) Patentinhaber: **CIBA-GEIGY AG, Patentabteilung Postfach, CH-4002 Basel (CH)**

(72) Erfinder: **Schupp, Thomas, Dr., Kurzrüttistrasse 11, CH-4313 Möhlin (CH)**
Erfinder: **Traxler, Peter, Dr., Gartenstrasse 24, CH-4123 Allschwil (CH)**
Erfinder: **Nüesch, Jakob, Prof. Dr., Im Lee 26, CH-4144 Arlesheim (CH)**

**EP 0 014 181 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Neue antibiotisch wirksame Verbindungen, ihre fermentative Herstellung und sie enthaltende pharmazeutische Präparate

Gegenstand der Erfindung ist eine Gruppe neuer antibiotisch wirksamer Verbindungen mit der Rifamycin-Grundstruktur, bestehend aus 3-Hydroxyrifamycin-S der Formel I, 3,31-Dihydroxyrifamycin-S der Formel II und 1-Desoxy-1-oxarifamycin-S der Formel III

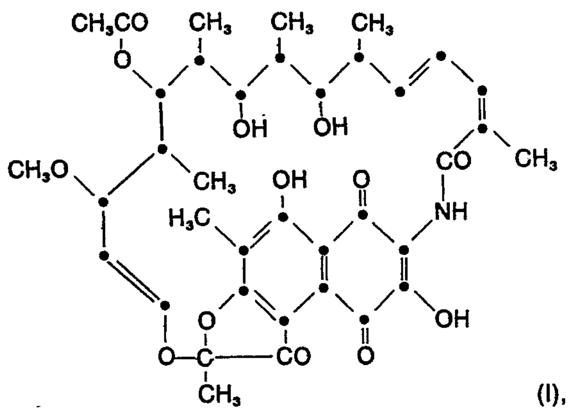

(I),

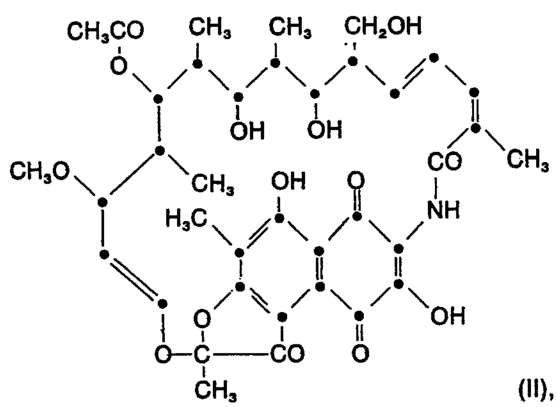

(II),

bzw.

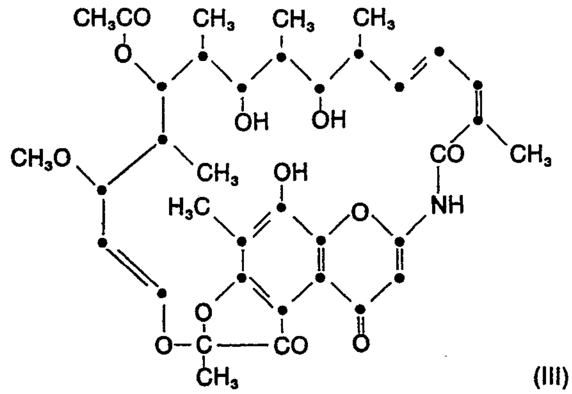

(III)

und ein Verfahren zur Herstellung derselben auf fermentativem Wege, sowie pharmazeutische Präparate enthaltend mindestens eine dieser Verbindungen als Wirkstoff, und ihre Herstellung.

Die erfindungsgemässen Verbindungen I–III zeichnen sich durch die folgenden Eigenschaften aus:

3-Hydroxyrifamycin-S (I) ist ein rotes amorphes Pulver, gut löslich in den meisten organischen Lösungsmitteln, unlöslich in Wasser und aliphatischen Kohlenwasserstoffen. – Das dünnschichtchromatographische Verhalten im Vergleich zur Rifamycin-S ist aus Tabelle 1 ersichtlich:

Tabelle 1
Rf-Werte von Verbindungen I–III und Rifamycin-S (Dünnschichtchromatographie auf Kieselgel-Platten 60 F 254, Hersteller: Merck, Darmstadt, BRD).

| | System 1 | System 2 | System 3 | System 4 |
|---|---|---|---|---|
| Rifamycin-S | 0,65 | 0,74 | 0,62 | 0,42 |
| I | 0,10 | 0,37 | 0,18 | 0,61 |
| II | 0,04 | 0,16 | 0,03 | 0,36 |
| III | 0,30 | 0,40 | 0,19 | 0,43 |

System 1: Chloroform-Methanol (9:1)
System 2: Chloroform-Methanol (6:1)
System 3: Äthylacetat-Aceton-Wasser (72:24:4)
System 4: Toluol-Aceton-Methanol (5:3:2)

Im Felddesorption-Massenspektrum weist 3-Hydroxyrifamycin-S (I) ein scharfes Maximum des Molekülions bei m/e = 711 auf, welches mit der Bruttoformel $C_{37}H_{45}NO_{13}$ übereinstimmt und damit sich lediglich um 16 Masseneinheiten, entsprechend einem Sauerstoffatom, von derjenigen des Rifamycin-S ($C_{37}H_{45}NO_{12}$) unterscheidet.

Im Infrarot-Spektrum (in $CH_2Cl_2$) von 3-Hydroxyrifamycin-S sind folgende für Rifamycine typischen Banden vorhanden: 3450 (OH), 3350 (Amid-NH), 2950, 1740 (5-Ring-Keton), 1715 (Acetat), 1685 (Amid I), 1640 und 1615 (Chinon-C=O), 1510 (Amid II), 1415, 1380, 1325, 1295, 1155, 1065 $cm^{-1}$. Das 360-MHz-NMR-Spektrum der Verbindung (s. Fig. 1) ist mit demjenigen von Rifamycin-S weitgehend identisch bis auf Signal bei 7,3 ppm, welches beim Rifamycin-S dem H(3)-Proton zugeschrieben wurde und bei der erfindungsgemässen Verbindung fehlt. Im Einklang damit findet man auch im $^{13}C$-NMR-Spektrum der erfindungsgemässen Verbindung das Signal des C(3)-Atoms erst bei 162 ppm, das heisst im Vergleich zum Rifamycin-S wesentlich nach tieferem Feld verschoben, was auf die Anwesenheit einer Hydroxylgruppe in dieser Stellung (Stellung 3) hindeutet. Alle diese physikalischen Daten stimmen mit der vorangehend angegebenen Strukturformel eindeutig überein. Zur Strukturermittlung, Nomenklatur und Numerierung der Rifamycine siehe V. Prelog und W. Oppolzer: Helv. Chim. Acta 56, 2279 (1973).

3,31-Dihydroxyrifamycin-S (II) ist ein rotes amorphes Pulver, gut löslich in den meisten organischen Lösungsmitteln, unlöslich in Wasser und aliphatischen Kohlenwasserstoffen. – Das dünnschichtchromatographische Verhalten im Vergleich zur Rifamycin-S ist aus Tabelle 1 ersichtlich:

Im Felddesorptions-Massenspektrum weist 3,31-Dihydroxyrifamycin-S (II) ein Molekülion bei m/e = 727 auf, welches mit der Bruttoformel $C_{37}H_{45}NO_{14}$ übereinstimmt und damit sich lediglich um 16 Masseneinheiten, entsprechend einem Sauerstoffatom, von derjenigen des 3-Hydroxyrifamycin-S (I) ($C_{37}H_{45}NO_{13}$) unterscheidet. Das Infrarot-Spektrum (in $CH_2Cl_2$) ist praktisch mit demjenigen von 3-Hydroxyrifamycin-S identisch. Im 360-MHz-NMR-Spektrum der Verbindung II, im Vergleich mit demjenigen von Rifamycin-S, fehlt wiederum das Signal bei 7,3 ppm, welches beim Rifamycin-S dem H(3)-Proton zugeschrieben wurde. Zusätzlich fehlt auch eines der Signale der sekundären Methylgruppen im Ansaring. Dafür tritt neu ein AB-System bei 3,5 ppm auf, welches der primären Hydroxygruppe an C(31) zugeschrieben werden kann. Aufgrund von Doppelresonanzversuchen kann die Kopplung des H(20)-Protons bei 2,5 ppm mit dem Vinylproton H(19) bei 5,95 ppm, dem H(21)-Proton bei 4 ppm, sowie den beiden H(31)-Protonen gezeigt werden.

Im $^{13}C$-NMR-Spektrum (s. Tabelle 2) wird C(31) bei 62,3 ppm gefunden. Dafür sind nur 7 Signale für Methylgruppen zwischen 8 bis 22 ppm vorhanden, verglichen mit 8 solchen Signalen in Rifamycin-S und 3-Hydroxyrifamycin-S (I).

Alle diese physikalischen Daten stimmen mit der postulierten Strukturformel überein.

1-Desoxy-1-oxarifamycin-S (III) ist ein schwach gelbes amorphes Pulver, gut löslich in den meisten organischen Lösungsmitteln, unlöslich in Wasser und aliphatischen Kohlenwasserstoffen. Das dünnschichtchromatographische Verhalten ist aus Tabelle 1 ersichtlich. 1-Desoxy-1-oxarifamycin-S (III) unterscheidet sich von Rifamycin-G [G. Lancini et al.; Journal of Antib. 29 (4), 466 (1976)] nur durch das Vorhandensein der Doppelbindung C(16)–C(17).

Im Massensepktrum weist die Verbindung III ein Molekülion bei m/e 683 auf (Rifamycin-G weist $M^+$ bei m/e 685 auf), welches mit der Bruttoformel von $C_{36}H_{45}NO_{12}$ übereinstimmt. Das IR-Spektrum ist praktisch identisch mit demjenigen von Rifamycin-G.

Im 100-MHz-NMR-Spektrum ($CDCl_3$) sind die Signale der C(16)–C(17), C(17)–C(18) und C(28)–C(29)-Doppelbindungen vorhanden. Das C(3)-Proton erscheint, wie bei Rifamycin-G, bei 7,2 ppm.

Auch das $^{13}C$-NMR-Spektrum (in $CD_3OD$) steht im Einklang mit der postulierten Struktur (s. Tabelle 2). Das Signal des C(1)-Atoms, bei Rifamycinen normalerweise bei 185 ppm auftretend, fehlt. Drastische Veränderungen gegenüber Rifamycin-S erfahren auch die Signale der aromatischen C-Atome (C2), C(3) und C(9).

Die erfindungsgemässen Rifamycin-Analogen I–III besitzen eine sehr gute, dem Rifamycin-S ähnliche antibiotische, insbesondere antimikrobielle Aktivität, vor allem gegen Gram-positive sowie Gram-negative Bakterien. Insbesondere in der antibiotischen Wirksamkeit gegen Gram-negative Bakterien, wie Escherichia coli und Pseudomonas sp., erweisen sie sich dem Rifamycin-S überlegen.

Erfindungsgemäss werden die Verbindungen I–III durch Isolierung aus einem Fermentationsgut erhalten, worin sie, je einzeln oder zusammen in beliebiger Kombination, durch die Züchtung von bestimmten, weiter unten näher charakterisierten Stämmen von Nocardia mediterranei entstehen.

Die Isolierung erfolgt auf physiko-chemischem Wege mittels an sich bekannter Trennungsmethoden, insbesondere auch Zentrifugieren, Filtrieren, Lösungsmittel-Extraktion, Fällung, Kristallisieren und Chromatographie, vor allem Adsorptions- und Verteilungschromatographie. – Bei einem typischen Isolierungsverfahren wird das Fermentationsgut (Kulturbrühe) vom Mycel durch Filtrieren, gegebenenfalls unter Anwendung von Filterhilfsmitteln, wie Kieselgur, befreit und das Kulturfiltrat der Extraktion mit einem organischen, mit Wasser begrenzt mischbaren Lösungsmittel, insbesondere mit Äthylacetat oder einem halogenierten aliphatischen Kohlenwasserstoff, wie Methylenchlorid, Chloroform oder Trichloräthylen, in einem diskontiuierlichen oder kontinuierlichen Verfahren, wie Gegenstromverfahren, unterworfen. Vorzugsweise wird das Kulturfiltrat vor der Extraktion durch Zugabe einer Säure, z.B. einer Mineralsäure, wie insbesondere Salzsäure, Schwefelsäure oder Phosphorsäure, oder auch einer stärkeren organischen Säure, wie Oxalsäure oder Zitronensäure, auf pH von etwa 2–3 gestellt. Aus der organischen Lösung werden flüchtige Anteile, vor allem Lösungsmittel, durch Abdampfen entfernt und der verbleibende Rückstand (roher Extrakt) weiterverarbeitet.

Zur Isolierung der erfindungsgemässen Verbindungen I–III und Entfernung der Begleitstoffe, z.B. anderer Metaboliten, insbesondere anderer Verbindungen mit Rifamycin-Grundstruktur, und gegebenenfalls auch zur Trennung individueller Verbindungen I–III voneinander, wird der rohe Extrakt vornehmlich durch Chromatographie, z.B. Säulenchromatographie, weiter gereinigt. Als Adsorbens ist z.B. Silicagel besonders geeignet, als Lösungsmittel eignet sich dabei z.B. Chloroform, dem 1–20% Methanol in allmählich ansteigender Menge beigemischt wird, wobei üblicherweise bei

einer Methanolkonzentration von 1–5% 3-Hydroxyrifamycin-S, bei einer Methanolkonzentration von 5–10% zuerst 1-Desoxy-1-oxarifamycin-S und dann auch 3,31-Dihydroxyrifamycin-S eluiert werden.

Das Reinigungsverfahren kann, wenn notwendig, wiederholt werden, gegebenenfalls unter Anwendung von anderen Adsorbentien und/oder Lösungsmittelsystemen. – Die Wirksamkeit der Reinigung kann in der üblichen Weise durch Dünnschichtchromatographie kontrolliert werden, wozu die oben angegebenen Bedingungen besonders vorteilhaft sind. Man kann auch biologische Testmethoden anwenden oder mit der Dünnschichtchromatographie kombinieren, z.B. die antibiotische Wirkung der einzelnen Fraktionen gegen einen geeigneten, vorzugsweise spezifisch empfindlichen, Mikroorganismus testieren. Als Testorganismus eignet sich Escherichia coli besonders gut, als Testmethode bewährt sich besonders die Kombination der dünnschichtchromatographischen Trennung und der bioautographischen Detektion. Dabei wird die entwickelte chromatographische Platte auf eine mit einem Testorganismus, z.B. Escherichia coli, bepflanzte Keimplatte abgedrückt, und der Wirkstoff durch die Bildung einer entsprechenden Hemmzone lokalisiert.

Zur Reindarstellung kleiner Mengen von 3-Hydroxyrifamycin-S kann man präparative Dünnschichtchromatographie mit Vorteil anwenden; geeignet sind dazu z.B. Kieselgel-Dünnschicht-Platten (wie die oben erwähnten Platten F 254) und ein Gemisch von Chloroform-Methanol (4 : 1 oder 6 : 1), oder Äthylacetat-Aceton-Wasser (72 : 24 : 4) oder Toluol-Aceton-Methanol (5 : 3 : 2) als Elutionsmittel. In analoger Weise werden auch 3,31-Dihydroxyrifamycin-S und 1-Desoxy-1-oxarifamycin-S rein erhalten, indem man dieselben Platten und Elutionssysteme verwendet. Die gewünschte Zone wird mechanisch abgetrennt, gesammelt und mit einem geeigneten Lösungsmittel, vorzugsweise Äthylacetat, zur Isolierung des reinen Produktes extrahiert. Dieses Reinigungsverfahren kann auch nach Bedarf unter Anwendung von verschiedenen Adsorbentien und/oder Lösungsmittelkombinationen wiederholt werden.

Ein 3-Hydroxyrifamycin-S, 3,31-Dihydroxyrifamycin-S und/oder 1-Desoxy-1-oxarifamycin-S enthaltendes Fermentationsgut, welches man zur Isolierung dieser Verbindungen in der oben beschriebenen Weise verwendet, erhält man erfindungsgemäss dadurch, dass man unter aeroben Bedingungen in einem flüssigen Medium einen Stamm von Nocardia mediterranei, der sich von Streptomyces mediterranei ATCC 13685 als Grundstamm ableitet und durch seine Fähigkeit, mindestens eine der erfindungsgemässen Verbindungen der Gruppe bestehend aus 3-Hydroxyrifamycin-S, 3,31-Dihydroxyrifamycin-S und 1-Desoxy-1-oxarifamycin-S zu produzieren, charakterisiert ist, züchtet. Unter diesen Stämmen sind gegen Streptomycin resistente und in bezug auf Aminosäuren autotrophe Rekombinantenstämme bevorzugt.

Als flüssiges Nährmedium verwendet man z.B. eine wässrige Lösung oder Suspension, welche mindestens eine Kohlenstoffquelle (die auch als Energiequelle dient) und mindestens eine Stickstoffquelle, und vorzugsweise auch Mineralstoffe enthält. Als Kohlenstoffquelle sind beispielsweise zu nennen: Glycerin, assimilierbare Kohlenhydrate, wie Cyclitole, z.B. Mannit, Polysaccharide, z.B. Stärke, Disaccharide, z.B. Lactose und Saccharose, und Monosaccharide, vor allem Glucose, sowie entsprechende Kohlenhydrat-haltige technische Rohstoffe, wie Zuckerrüben- oder Zuckerrohrmelasse. Als Stickstoffquelle seien genannt: Aminosäure, insbesondere die in der Natur vorkommenden α-Aminosäuren, Peptide sowie Proteine und deren Abbauprodukte, wie Peptone und Tryptone, aber auch Ammoniumsalze und Nitrate sowie entsprechende technische stickstoffhaltige Rohstoffe, wie Fleischextrakte, Kaseinhydrolysat sowie Hefeautolysat und -extrakt. Es kommen auch gemischte technische C- und N-Quellen in Betracht, wie verschiedene Pflanzensamen, die in Form von wässrigen Auszügen, Mehl oder Maische von Bohnen, z.B. Sojabohnen, Getreidekörnern, z.B. Weizen und insbesondere Mais («Cornsteep liquor»); ferner auch Baumwollsamen, sowie auch Malzextrakt verwendet werden. Neben Ammoniumsalzen und Nitraten kann das Nährmedium als anorganische Salze, Chloride, Carbonate, Sulphate und insbesondere Phosphate von Alkali- und Erdalkalimetallen, sowie von Spurenelementen, wie Magnesium, Eisen, Zink und Mangan, enthalten.

Das Nährmedium wird in der konventionellen Weise zubereitet, sterilisiert und mit einer Kultur des Produktionsstammes unter Einhalten der üblichen Massnahmen eingeimpft. Die Züchtung erfolgt unter aeroben Bedingungen, z.B. in ruhender Oberflächenkultur oder vorzugsweise in submerser Kultur, die durch Schütteln und/oder Rühren mit Sauerstoff, üblicherweise in Form von Luftsauerstoff, versorgt wird, z.B. in Schüttelkolben oder Fermentern bekannter Konstruktion. Als Temperatur eignet sich eine solche zwischen etwa 20 bis etwa 35 °C, vorzugsweise etwa 22 bis 30 °C und insbesondere von etwa 28 °C. Die Züchtung führt man bei einem pH von etwa 5,0–9,0, vorzugsweise im Bereich von etwa 6,0–8,0 und insbesondere in der Nähe des Neutralpunktes, aus. Üblicherweise ist kein Nachstellen vom pH während der Fermentation erforderlich. Unter diesen Bedingungen wird im Verlaufe von etwa 3 bis 14, üblicherweise etwa 7 Tagen, das Maximum von 3-Hydroxyrifamycin-S produziert und ins Nährmedium ausgeschieden. Bei grösseren Ansätzen kultiviert man stufenweise, wobei man zunächst eine oder mehrere Vorkulturen in flüssigem Nährmedium während einer kürzeren Zeit, z.B. etwa 2–3 Tagen, heranwachsen lässt und in eine jeweils grössere z.B. zwanzigfache, Menge Nährmedium bis zum gewünschten Produktionsvolumen überimpft.

Der erfindungsgemässe Produktionsmikroorganismus, d.h. ein mindestens eine der Verbindungen der Gruppe bestehend aus 3-Hydroxyrifamycin-S, 3,31-Dihydroxyrifamycin-S und 1-Desoxy-1-oxarifamycin-S produzierender Stamm von Nocardia mediterranei, kann erhalten werden, indem man einen zur Überprüfung geeigneten Mutanten- oder Rekombinantenstamm von Nocardia mediterranei, der sich von Streptomyces mediterranei ATCC 13685 als Grundstamm ableitet, züchtet und das erhaltene Fermentationsgut auf die Gegenwart von 3-Hydroxyrifamycin-S 3,31-Dihydroxyrifamycin-S und/oder 1-Desoxy-1-oxarifamycin-S prüft, z.B. wie unten beschrieben ist. – Ein vorteilhafter Rekombinantenstamm wird z.B. durch Kreuzen und selektiven Genaustausch zwischen zwei Nocardia mediterranei-Mutanten A und B, die sich von Streptomyces mediterranei ATCC 13 685 als Grundstamm ableiten [vgl. T. Schupp et al.: Journal of Bacteriology 121, 128–136 (1975)] erhalten. (Der Grundstamm wurde unter der ursprünglichen taxonomischen Bezeichnung Streptomyces mediterranei (Margalith und Bretta) in der Sammlung ATCC unter der Nummer 13 685 hinterlegt. Später wurde aufgrund weiterer Untersuchungen diese Bezeichnung revidiert und der Name Nocardia mediterranei (Thieman et al.) ATCC 13 685 vorgeschlagen. Hier wird die ursprüngliche Sammlungsbezeichnung verwendet.)

Die Mutanten-Stämme A und B ihrerseits werden in an sich bekannter Weise durch die mutagene Einwirkung von ultraviolettem Licht oder von N-Methyl-N'-nitro-N-nitrosoguanidin auf die Mycelsuspension des Grundstammes Streptomyces mediterranei ATCC 13 685 erhalten und durch Selektion nach ihren spezifischen Eigenschaften isoliert. Der Mutanten-Stamm A produziert hauptsächlich Rifamycin-B, womit er dem Grundstamm ähnlich ist, im Gegensatz zu diesem besitzt er zwar einerseits eine 50fach höhere Resistenz gegen Streptomycin, anderseits ist er aber in bezug auf Cystein, Lysin und Leucin auxotroph, d.h. weist ein Wachstumsbedürfnis für diese drei Aminosäuren auf. Der Mutanten-Stamm B zeichnet sich in erster Linie dadurch aus, dass er Streptomycin-empfindlich ist, und insbesondere, dass er kein Rifamycin-B mehr produziert, sondern lediglich ein Zwischenprodukt der Rifamycin-Synthese, das Rifamycin-W [vgl. R.J. White et al.: Proc. Nat. Acad. Sci. USA 71, 3260–3269 (1974)]. Die Rekombination wird in an sich bekannter Weise durch gemeinsame Züchtung beider Mutanten-Stämme durchgeführt und der Rekombinantenstamm nach seinen spezifischen Eigenschaften durch Selektion nach bekannten Methoden isoliert; zur Methodik vgl. z.B. die obgenannte Publikation von T. Schupp et al.

Eine für die Isolierung des gewünschten Rekombinanten-Stammes besonders wichtige Operation ist gemeinsames Bebrüten beider Mutanten A und B auf einem Vollmedium, z.B. Kulturmedium Nr. 2, und anschliessende Züchtung der erhaltenen Mischkultur auf einem selektiven Minimalmedium, z.B. dem weiter unten beschriebenen Kulturmedium Nr. 3. Für ein solches Minimalmedium ist besonders charakteristisch, dass es nebst einer möglichst einfachen Kohlenstoff- und Energiequelle, wie einem einfachen Zucker, z.B. Glucose, lediglich anorganische Salze enthält, insbesondere ein anorganisches Ammoniumsalz als die einzige Stickstoffquelle. Auf einem solchen selektiven Minimalmedium kann weder der auxotrophe Stamm A wachsen, da ihm die Aminosäuren Leucin, Cystein und Lysin fehlen, noch der Stamm B, da er durch 0,025 g/l Streptomycin gehemmt wird. Somit findet man nach Ablauf der Inkubationszeit nur Kolonien von Rekombinantenstämmen auf dem verwendeten Medium, die durch Genaustausch vom Stamm A die erhöhte Resistenz gegen Streptomycin und vom Stamm B die Fähigkeit, ohne die Aminosäuren Leucin, Cystein und Lysin zu wachsen, übertragen bekommen haben. Aus mehreren auf diese Weise selektionierten Rekombinantenstämmen erhält man die gewünschte, die erfindungsgemässen Rifamycin-Analoga-produzierende, Kultur durch separate Züchtung einzelner Kolonien und Prüfung auf die Gegenwart von 3-Hydroxyrifamycin-S, 3,31-Dihydroxyrifamycin-S und/oder 1-Desoxy-1-oxarifamycin-S unter den Metaboliten. Diese Prüfung erfolgt vornehmlich durch Extraktion des Kulturfiltrates mit einem organischen Lösungsmittel, wie oben beschrieben wurde, und die kombinierte dünnschichtchromatographische Trennung und bioautographische Detektion des gewünschten Antibiotikums, insbesondere unter Anwendung von Escherichia coli als Testmikroorganismus.

Als ein besonders geeigneter, typischer Mutanten-Stamm A hat sich der Mutanten-Stamm Nocardia mediterranei T 104, als Mutanten-Stamm B der Mutanten-Stamm Nocardia mediterranei T 191 (beide abgeleitet vom Stamm Streptomyces mediterranei ATCC 13 685 als Grundstamm) besonders bewährt. Diese spezifischen Mutanten-Stämme besitzen in vollem Umfang die für die Mutanten-Stämme A und B charakteristischen Eigenschaften. Durch Rekombination der genannten Stämme T 104 und T 191 und selektive Isolierung, wie oben beschrieben, wird insbesondere der Rekombinanten-Stamm Nocardia mediterranei R 21 erhalten, welcher unter der Bezeichnung Nocardia mediterranei DSM 1415 bei der Deutschen Sammlung von Mikroorganismen, Göttingen, Bundesrepublik Deutschland, am 29.12.1978 hinterlegt wurde. (Unter der Bezeichnung T 104, T 191 und R 21 sind die Nocardia-Stämme in der Sammlung der Mikroorganismen der Fa. Ciba-Geigy AG, 4002 Basel, Schweiz, aufbewahrt.) Der Rekombinantenstamm R 21 weist völlig die oben angegebenen charakteristischen Eigenschaften eines bevorzugten erfindungsgemässen 3-Hydroxyrifamycin-S-produzierenden Rekombinanten-Stammes auf, indem er diese Verbindung als Hauptprodukt, 3,31-Dihydroxyrifamycin-S und 1-Desoxy-1-oxarifamycin-S dann als wesentliche Begleitstoffe produziert, in bezug auf Aminosäuren autotroph ist und im Vergleich mit dem Grundstamm ATCC 13 685 50mal resistenter gegen Streptomycin ist. In den sonstigen Merkmalen, insbesondere in der Morphologie, lassen sich keine charakteristischen Unterschiede gegenüber dem Grundstamm ATCC 13 685 feststellen.

Die Erfindung umfasst ebenfalls die Verwendung von 3-Hydroxyrifamycin-S, 3,31-Dihydroxyrifamycin-S und 1-Desoxy-1-oxarifamycin S je allein oder in Kombination miteinander oder mit anderen Antibiotika, insbesondere denjenigen vom Rifamycin-Typ, als Antibiotikum zur Bekämpfung von Infektionen, die durch Bakterien, z.B. die genannten, hervorgerufen werden, ausserhalb des menschlichen Körpers, z.B. als Desinfektionsmittel. Bei einer alternativen Verwendung, d.h. als Heilmittel, wird der genannte Wirkstoff vorzugsweise in Form eines pharmazeutischen Präparats zusammen mit mindestens einem konventionellen pharmazeutischen Träger oder Hilfsstoff einem Warmblüter, vor allem Menschen verabreicht.

Zwecks Herstellung pharmazeutischer Präparate kann jede einzelne der genannten erfindungsgemässen Verbindungen, vor allem 3-Hydroxyrifamycin-S, mit einem für die topische, enterale oder parenterale Applikation geeigneten anorganischen oder organischen Trägermaterial vermischt werden. Für dasselbe kommen solche Stoffe in Betracht, die mit der neuen Verbindung nicht reagieren, wie z.B. Gelatine, Milchzucker, Stärke, Magnesiumstearat, pflanzliche Öle, Benzylalkohol, oder andere Arzneimittelträger. Die pharmazeutischen Präparate können z.B. als Tabletten, Dragées, Pulver, Suppositorien, oder in flüssiger Form als Lösungen, Suspensionen, Emulsionen, Cremen oder Salben vorliegen. Gegebenenfalls sind sie sterilisiert und/oder enthalten Hilfsstoffe wie Konservierungsmittel, Stabilisierungs-, Netz- oder Emulgiermittel. Sie können auch noch andere therapeutisch wertvolle Stoffe enthalten. Auch die Desinfektionsmittel können mit geeigneten Trägerstoffen, wie bekannt, vermischt werden.

Die Dosierung der Wirkstoffe (3-Hydroxyrifamycin-S, 3,31-Dihydroxyrifamycin-S und 1-Desoxy-1-oxarifamycin-S erfolgt im Prinzip analog derjenigen von anerkannten Antibiotika vom Rifamycin-Typ, insbesondere dann der Dosierung von Rifamycin-S; sie hängt jedoch auch einerseits von Spezies, Körpergewicht, Alter und individuellem Zustand des Warmblüters, anderseits von der Applikationsweise und insbesondere von der jeweiligen Empfindlichkeit des Krankheitserregers ab.

Die Erfindung betrifft auch eine Methode zur Tötung oder Wachstumsbehinderung eines gegen mindestens eine der erfindungsgemässen Verbindungen I–III, wie 3-Hydroxyrifamycin-S, empfindlichen Mikroorganismus, welche durch die Behandlung dieses Mikroorganismus oder eines durch diesen Mikroorganismus infizierten Medium mit einer antimikrobiell wirksamen Dosis einer der erfindungsgemässen Verbindungen I–III, insbesondere 3-Hydroxyrifamycin-S, charakterisiert ist. – Unter der Bezeichnung «eine antimikrobiell wirksame Dosis» ist eine solche Menge des Wirkstoffes zu verstehen, die zu einer wirkungsvollen Inhibition des betreffenden zu behandelnden Mikroorganismus ausreicht.

Die nachfolgenden Beispiele illustrieren die oben beschriebene Erfindung; sie sollen jedoch diese in ihrem Umfang in keiner Weise einschränken. Temperaturen werden in Celsiusgraden angegeben. Die Zusammensetzung von Lösungsmittelgemischen ist im Volumenverhältnis angegeben.

Es werden die folgenden Nährmedien verwendet:

Kulturmedium Nr. 1

| | |
|---|---|
| Glucose | 22 g |
| Rindfleischextrakt | 5 g |
| Pepton | 5 g |
| Bierhefeextrakt | 5 g |
| Enzymatisches Kaseinhydrolysat | 3 g |
| NaCl | 1,5 g |

Destilliertes Wasser auffüllen auf 1000 ml pH-Wert 7,0, Sterilisation 20 Minuten bei 120 °C.

Kulturmedium Nr. 2

| | |
|---|---|
| Bierhefeextrakt | 4 g |
| Malzextrakt | 10 g |
| Glucose | 4 g |
| Agar | 20 g |

Destilliertes Wasser auffüllen auf 1000 ml pH mit KOH vor Sterilisation auf 7,3 einstellen, Sterilisation 20 Minuten bei 120 °C.

Kulturmedium Nr. 3

| | |
|---|---|
| $K_2HPO_4$ | 1,0 g |
| $(Na_4)_2SO_4$ | 2,0 g |
| $MgSO_4 \cdot 7H_2O$ | 1,0 g |
| NaCl | 1,0 g |
| $CaCO_3$ | 1,0 g |
| $FeSO_4 \cdot 7H_2O$ | 0,001 g |
| $MnCl_2 \cdot 4H_2O$ | 0,001 g |
| $ZnSO_4 \cdot 7H_2O$ | 0,001 g |
| Agar | 20,0 g |

Destilliertes Wasser auffüllen auf 1000 ml Sterilisation 20 Minuten bei 120 °C.

Zur erhaltenen, noch heissen Lösung wird unter sterilen Bedingungen zugefügt: a) 20 ml einer Glucose-Lösung, zubereitet durch Lösen von 50 g Glucose in destilliertem Wasser, Auffüllen auf 100 ml und Sterilisieren 20 Minuten bei 120 °C, und b) 5 ml einer Streptomycin-Lösung, zubereitet durch Lösen von 0,5 g Streptomycin in 100 ml destilliertem Wasser und Sterilfiltrieren.

Kulturmedium Nr. 4

| | |
|---|---|
| Bierhefeextrakt | 3 g |
| Pepton | 5 g |
| Malzextrakt | 3 g |
| Saccharose | 10 g |
| Agar | 20 g |
| Dest. Wasser auffüllen auf | 1000 ml |

Sterilisieren 20 Minuten bei 120 °C.

Beispiel 1

(Isolierung des Rekombinantenstammes R 21)

Die beiden zu kreuzenden Mutanten-Stämme T 104 und T 191 Nocardia mediterranei werden separat in je 40 ml des Kulturmediums Nr. 1 während 3 Tagen auf einem Rotationsschüttler bei 250 UpM und 28 °C bebrütet. Je 2 ml Kulturlösung der beiden Stämme werden in ein steriles Reagenzglas zusammengegeben und heftig gemischt. Mit 0,2 ml dieser Mischung wird ein Schrägagar (= Kulturmedium Nr. 2) beimpft.

Die Schrägagarkultur wird 6 Tage bei 28 °C bebrütet. (In dieser Phase findet der Genaustausch zwischen den beiden Stämmen T 104 und T 191 statt.) Zur Schrägagarkultur wird nach Ablauf der Bebrütungszeit so viel destilliertes Wasser steril zugegeben, dass das auf der Agaroberfläche anhaftende Mycel durch Kratzen mit einer Öse in Suspension gebracht wird. Diese Suspension wird in ein steriles Reagenzglas gefüllt und mit sterilen Quarzsteinchen von 2–3 mm Durchmesser während 2 Minuten heftig geschüttelt. Die so erhaltene Suspension kurzer Mycelstücke wird dann durch zweimaliges Zentrifugieren und Resuspendieren in destilliertem Wasser gewaschen und 100fach mit destilliertem Wasser verdünnt. Je 0,1 ml dieser verdünnten Suspension werden auf Petrischalen mit einem selektiven Minimalmedium (= Kulturmedium Nr. 3) plattiert.

Die beimpften Petrischalen werden bei 28 °C während 10–14 Tagen inkubiert, wodurch nur das Wachstum der autotrophen, gegen Streptomycin resistenten Rekombinanten ermöglicht ist und die ursprünglichen Mutanten gehemmt bleiben. Zur Isolierung 3-Hydroxyrifamycin-S-produzierender Rekombinanten-Stämme wird von jeder angewachsenen Kolonie je ein Schrägagar des Kulturmediums Nr. 4 beimpft und 8 Tage bei 28 °C bebrütet. Von diesen Kulturen wird je eine Öse voll Mycel in 20 ml der Kulturlösung Nr. 1 in kleine Erlenmeyerkolben geimpft, und diese werden bei 28 °C auf einem Rotationsschüttler bei 250 UpM gehalten. Nach 7 Tagen Bebrütung werden die Kulturlösungen durch Papierfilter filtriert, das erhaltene Kulturfiltrat mit Salzsäure auf pH 2,5 angesäuert und mit gleichem Volumen Methylenchlorid extrahiert. Der so erhaltene Extrakt wird auf $^1/_{20}$ eingeengt und durch Dünnschichtchromatographie auf Kieselgel 60 F 254 Platten (Merck) mit dem Laufsystem Chloroform-Methanol (4 : 1) entwickeln. Durch Abdrücken dieser entwickelten Dünnschichtplatten auf Escherichia coli-Keimplatten (Bioautogramm) zeigt sich durch eine Hemmzone (Rf 0,45), welcher der untersuchten Extrakte 3-Hydroxyrifamycin-S enthält und somit, welcher der untersuchten Rekombinantenstämme 3-Hydroxyrifamycin-S produziert. Der auf diese Art isolierte Rekombinantenstamm R 21 wurde bei der Deutschen Sammlung von Mikroorganismen, wie oben angegeben, unter der Bezeichnung Nocardia mediterranei DSM 1415 hinterlegt.

Beispiel 2

Fermentative Herstellung von 3-Hydroxyrifamycin-S (I) und/oder Verbindungen II und III.

– Der Stamm Nocardia mediterranei R 21 wird in einer Schrägagarkultur (Kulturmedium Nr. 4) während 7–8 Tagen bei 28 °C gezüchtet. Mit dem Mycel dieser Kultur werden 5 Erlenmeyerkolben mit je 40 ml Kulturmedium Nr. 1 beimpft. Die Kolben werden während 72 Stunden bei 28 °C und 250 UpM auf dem Rotationsschüttler gehalten. Nach dem 72stündigem Wachstum werden 150 ml vegetative Kultur zum Beimpfen von 3 Liter Kulturmedium Nr. 1 verwendet. Die Fermentation wird mit jeweils 40 ml Medium in 200 ml Erlenmeyerkolben durchgeführt und läuft auf dem Rotationsschüttler bei 250 UpM und 28 °C während 7 Tagen ab.

Beispiel 3

[Isolierung und Reindarstellung von 3-Hydroxyrifamycin-S (I), 3,31-Dihydroxyrifamycin-S (II) und 1-Desoxy-1-oxarifamycin-S (III). – Das nach Beispiel 2 erhaltene Fermentationsgut (3 Liter) wird über Kieselgur filtriert, das Filtrat mit 1-N Salzsäure auf pH 2,5 gestellt und dreimal mit Chloroform extrahiert. Die wässrige Phase wird verworfen, die organische im Vakuum konzentriert. Der resultierende dunkel gefärbte rohe Extrakt (2,02 g) wird in 200 ml Methanol gelöst, mit 10 g Silicagel vermischt und zur Trockne eingedampft. Mit dem getrockneten pulverigen Rückstand wird eine chromatographische Säule (Durchmesser = 1 cm, Höhe = 40 cm) von 100 g Silicagel (Merck, Korngrösse 65–200 nm) überschichtet. Die Elution erfolgt mit Lösungsmittelgemischen von Chloroform mit stufenweise ansteigender Konzentration (1 bis 20%) Methanol. Einzelne Fraktionen (zu je 25 ml) werden im Wasserstrahl-Vakuum eingedampft und im Hochvakuum getrocknet. Die Fraktionen werden aufgrund dünnschichtchromatographischer (und gegebenenfalls auch bioautographische) Kontrolle vereinigt; der Hauptanteil von 3-Hydroxyrifamycin-S (I) befindet sich in Fraktionen mit 1–5% Methanol, 1-Desoxy-1-oxarifamycin-S (III) und 3,31-Dihydroxyrifamycin-S (II) dann in denjenigen mit 5–10% Methanol.

Zur weiteren Reinigung (z. B. für analytische Zwecke) werden rohe Produkte I, II und III auf Dünnschichtplatten (Silicagel 60 F 254 – siehe oben) mit Gemischen von Chloroform-Methanol (4 : 1) oder (6 : 1), Äthylacetat-Aceton-Wasser (72 : 24 : 4) oder Toluol-Aceton-Methanol (5 : 3 : 2) chromatographiert. Die gewünschte Zone wird mechanisch abgetrennt und mit Äthylacetat extrahiert. Der Extrakt wird mit einer verdünnten Zitronensäure-Lösung gewaschen, getrocknet und eingedampft. Es resultieren reines 3-Hydroxyrifamycin-S und 3,31-Dihydroxyrifamycin-S, je in Form eines roten amorphen Pulvers, sowie 1-Desoxy-1-oxarifamycin-S als schwach gelbes Pulver. Jede der Verbindungen ist in Methanol, Äthanol und anderen Niederalkanolen, Aceton, Dimethylsulfoxid, Äthylacetat, Dioxan, Tetrahydrofuran, Äther; Dimethylformamid, Chloroform, Methylenchlorid und anderen ähnlichen chlorierten niederaliphatischen Kohlenwasserstoffen gut löslich, in Pentan, Hexan und ähnlichen aliphatischen Kohlenwasserstoffen, sowie im Wasser, praktisch unlöslich ist. Das dünnschichtchromatographische Verhalten, insbesondere im Vergleich zu Rifamycin-S, ist oben in Tabelle 1 angegeben.

3-Hydroxyrifamycin-S (I)

Physikalische Kenndaten: Felddesorptions-Massenspektrum:

$M^+$ bei m/e 711 ($C_{37}H_{45}NO_{13}$); UV-Spektrum:
a) in Äthanol: $\lambda_{max}$ ($\varepsilon$) 230 (29 600), 260 sh, 305 sh, 335 sh, 602 (240);
b) in 0,01-N HCl: $\lambda_{max}$ ($\varepsilon$) 267 (14 900), 305 sh, 342 (4600);
c) in 0,01-N NaOH: $\lambda_{max}$ ($\varepsilon$) 245 sh, 308 (24 500), 440 (5200).
Infrarot-Spektrum (in $CH_2CL_2$): Banden bei 3450, 3350, 2950, 1740, 1715, 1685, 1640, 1615, 1510, 1415, 1380, 1325, 1295, 1155 und 1065 $cm^{-1}$; 360 MHz-NMR-Spektrum (in $CDCl_3$): siehe Fig. 1.
$^{13}C$-NMR-Spektrum (in $CDCl_3$): siehe Tabelle 2.

3,31-Dihydroxyrifamycin-S (II)

Physikalische Kenndaten: Felddesorptions-Massenspektrum:
$M^+$ bei m/e 727 ($C_{37}H_{45}NO_{14}$); Infrarot-Spektrum (in $CH_2Cl_2$). Banden bei 3450, 3350, 2950, 1740, 1715, 1685, 1640, 1615, 1510, 1410, 1385, 1320, 1290, 1155 und 1065 $cm^{-1}$; 360 MHz-NMR-Spektrum (in $CDCl_3$) (nur die wichtigsten Signale):

| ppm | Zuordnung | ppm | Zuordnung |
|---|---|---|---|
| 0,1; 0,6; 1,0 | $C\underline{H}_3$(32, 33, 34) | 3,95 | H(21) |
| | | 4,6 | H(25) |
| 1,7 | $C\underline{H}_3$(13) | 5,0 | H(28) |
| 1,95 | $CO-C\underline{H}_3$ | 5,95 | H(19) |
| 2,0 | $C\underline{H}_3$(30) | 6,1 | H(29) |
| 2,25 | $C\underline{H}_3$(14) | 6,3 | H(17) |
| 2,45 | H(20) | 6,45 | H(18) |
| 2,95 | H(23) | 8,4 | $N\underline{H}-CO$ |
| 3,05 | $O-C\underline{H}_3$ | | |
| 3,3 | H(27) | 12,7 | $-O\underline{H}$(8) |
| 3,5 | H(31) | | |

$^{13}C$-NMR-Spektrum (in $CDCl_3$): siehe Tabelle 2.

1-Desoxy-1-oxarifamycin-S (III)

Physikalische Kenndaten: Massenspektrum:
$M^+$ bei m/e 683 ($C_{36}H_{45}NO_{12}$); IR-Spektrum (in $CH_2Cl_2$): Banden bei 3450, 2950, 1740 sh, 1710, 1650, 1595, 1520, 1245, 1155, 1060 $cm^{-1}$. 100 MHz-NMR-Spektrum ($CDCl_3$)

| ppm | Zuordnung | ppm | Zuordnung |
|---|---|---|---|
| 0,6–1,1 | $3\text{-}CH-C\underline{H}_3$ | 5,24 | H(28) |
| 1,72 | $C\underline{H}_3$(13) | 5,72 | H(19) |
| 2,0 | $CO-C\underline{H}_3$; $C\underline{H}_3$(3,0) | 6,24 | H(29) |
| 2,26 | $CH_3$(14) | 6,32 | H(17) |
| 3,1 | O–CH 3 | 6,4 | H(18) |
| 4,7 | H(25) | 7,2 | H(3) |

$^{13}C$-NMR-Spektrum (in $CD_3OD$): siehe Tabelle 2.

Tabelle 2: $^{13}C$-NMR-Daten von Rifamycin-S, 3-Hydroxyrifamycin-S (I), 3,31-Dihydroxy-rifamycin-S (II) und 1-Desoxy-1-oxarifamycin-S (III)

| Zuordnung C-Atom | Rifamycin-S ($CDCl_3$) | I ($CDCl_3$) | II ($CDCl_3$) | III ($CD_3OD$) |
|---|---|---|---|---|
| 1 | 184,5 | 184,9 | 185,0 | – |
| 2 | 139,4 | 118,4 | 118,3 | 171,6 |
| 3 | 117,4 | 146,8 | 147,1 | 99,2 |
| 4 | 181,6 | 177,2 | 177,3 | 178,8 |
| 5 | 111,2 | 111,0 | 111,0 | 109,1 |
| 6 | 166,5 | 171,9 | 172,0 | 155,9 |

Tabelle 2: Fortsetzung

| Zuordnung C-Atom | Rifamycin-S ($CDCl_3$) | I ($CDCl_3$) | II ($CDCl_3$) | III ($CD_3OD$) |
|---|---|---|---|---|
| 7 | 115,7 | 116,8 | 116,7 | 115,4 |
| 8 | 172,2 | 166,4 | 166,5 | 156,8 |
| 9 | 111,0 | 109,8 | 109,7 | 143,5 |
| 10 | 131,3 | 129,1 | 129,1 | 120,0 |
| 11 | 191,1 | 191,8 | 192,0 | 194,1 |
| 12 | 108,6 | 108,1 | 107,9 | 106,4 |
| 13 | 22,2 | 22,0 | 21,8 | 22,2 |
| 14 | 7,4 | 7,6 | 7,7 | 8,5 |
| 15 | 169,0 | 171,4 | 171,2 | 169,8 |
| 16 | 131,0 | 128,8 | 129,5 | 131,4 |
| 17 | 133,2 | 135,3 | 135,0 | 134,7 |
| 18 | 124,4 | 123,9 | 127,0 | 125,3 |
| 19 | 142,4 | 143,0 | 138,2 | 142,6 |
| 20 | 39,2 | 38,8 | 46,5 | 40,2 |
| 21 | 73,6 | 73,2 | 69,2 | 74,0 |
| 22 | 33,0 | 32,8 | 32,9 | 34,0 |
| 23 | 77,7 | 77,4 | 77,5 | 77,7 |
| 24 | 37,4 | 37,6 | 37,6 | 38,9 |
| 26 | 37,4 | 37,4 | 37,8 | 38,4 |
| 25 | 73,6 | 73,5 | 73,6 | 74,0 |
| 27 | 81,9 | 81,3 | 80,5 | 82,7 |
| 28 | 115,7 | 115,6 | 116,5 | 118,3 |
| 29 | 145,3 | 144,7 | 144,0 | 146,1 |
| 30 | 20,0 | 20,1 | 20,1 | 20,2 |
| 31 | 16,8 | 16,9 | 62,3 | 17,3 |
| 32 | 11,4 | 11,3 | 11,1 | 11,4 |
| 34 | 11,4 | 11,6 | 11,8 | 12,4 |
| 33 | 8,8 | 8,9 | 8,7 | 9,4 |
| 35 | 172,6 | 173,0 | 173,0 | 172,9 |
| 36 | 20,9 | 21,0 | 20,9 | 21,1 |
| 37 | 56,8 | 56,8 | 56,9 | 56,5 |

**Patentansprüche (für die Vertragsstaaten: BE, CH, DE, FR, GB, IT, LU, NL, SE**

1. Verfahren zur Herstellung von 3-Hydroxyrifamycin-S, 3,31-Dihydroxyrifamycin-S und 1-Desoxy-1-oxarifamycin-S, gekennzeichnet durch die Züchtung eines Stammes von Nocardia mediterranei, welcher sich von Streptomyces mediterranei ATCC 13 685 als Grundstamm ableitet und durch die Fähigkeit, mindestens eine der obgenannten Verbindungen zu produzieren, charakterisiert ist, unter aeroben Bedingungen in flüssigem Nährmedium und durch die Isolierung mindestens eines der obgenannten Endstoffe aus dem erhaltenen Fermentationsgut.

2. Verfahren zur Herstellung eines 3-Hydroxyrifamycin-S, 3,31-Dihydroxyrifamycin-S und/oder 1-Desoxy-1-oxarifamycin-S enthaltenden Fermentationsgutes, gekennzeichnet durch die Züchtung eines Stammes von Nocardia mediterranei, der von Streptomyces mediterranei ATCC 13 685 als Grundstamm abgeleitet und durch die Fähigkeit, mindestens eine der genannten Verbindungen zu produzieren, charakterisiert ist, unter aeroben Bedingungen in flüssigem Nährmedium.

3. Verfahren gemäss Anspruch 1 oder 2, dadurch gekennzeichnet, dass man einen, mindestens eine der genannten Verbindungen produzierenden, in bezug auf Aminosäuren autotrophen und gegen Streptomycin resistenten Rekombinantenstamm von Nocardia mediterranei, abgeleitet von Streptomyces mediterranei ATCC 13 685 als Grundstamm, unter aeroben Bedingungen in flüssigem Nährmedium züchtet.

4. Verfahren gemäss Anspruch 3, dadurch gekennzeichnet, dass man den Rekombinantenstamm Nocardia mediterranei DMS 1415 unter aeroben Bedingungen in flüssigem Nährmedium züchtet.

5. Verfahren gemäss einem der Ansprüche 1 bis 4, dadurch gekennzeichnet, dass man in einem wässrigen Nährmedium enthaltend eine Kohlenstoffquelle, eine Stickstoffquelle und Mineralsalze züchtet.

6. Kombinantenstamm Nocardia mediterranei DMS 1415.

7. 3-Hydroxyrifamycin-S.

8. 3,31-Dihydroxyrifamycin-S.

9. 1-Desoxy-1-oxarifamycin-S.

10. 3-Hydroxyrifamycin-S, 3,31-Dihydroxyrifamycin-S und/oder 1-Desoxy-1-oxarifamycin-S zur Verwendung als Antibiotikum.

11. Verwendung von 3-Hydroxyrifamycin-S, 3,31-Dihydroxyrifamycin-S und/oder 1-Desoxy-1-

oxarifamycin-S je einzeln oder zusammen in beliebiger Kombination zur Bekämpfung von bakteriellen Infektionen ausserhalb des menschlichen und tierischen Körpers.

12. Pharmazeutische Präparate enthaltend mindestens eine der in den Ansprüchen 7 bis 9 angegebenen Verbindungen.

**Patentansprüche (für den Vertragsstaat AT)**

1. Verfahren zur Herstellung von 3-Hydroxyrifamycin-S, 3,31-Dihydroxyrifamycin-S und 1-Desoxy-1-oxarifamycin-S, gekennzeichnet durch die Züchtung eines Stammes von Nocardia mediterranei, welcher sich von Streptomyces mediterranei ATCC 13 685 als Grundstamm ableitet und durch die Fähigkeit, mindestens eine der obgenannten Verbindungen zu produzieren, charakterisiert ist, unter aeroben Bedingungen in flüssigem Nährmedium und durch die Isolierung mindestens eines der obgenannten Endstoffe aus dem erhaltenen Fermentationsgut.

2. Verfahren zur Herstellung eines 3-Hydroxyrifamycin-S, 3,31-Dihydroxyrifamycin-S und/oder 1-Desoxy-1-oxarifamycin-S enthaltenden Fermentationsgutes, gekennzeichnet durch die Züchtung eines Stammes von Nocardia mediterranei, der von Streptomyces mediterranei ATCC 13 685 als Grundstamm abgeleitet und durch die Fähigkeit, mindestens eine der genannten Verbindungen zu produzieren, charakterisiert ist, unter aeroben Bedingungen in flüssigem Nährmedium.

3. Verfahren gemäss Anspruch 1 oder 2, dadurch gekennzeichnet, dass man einen, mindestens eine der genannten Verbindungen produzierenden, in bezug auf Aminosäuren autotrophen und gegen Streptomycin resistenten Rekombinantenstamm von Nocardia mediterranei, abgeleitet von Streptomyces mediterranei ATCC 13 685 als Grundstamm, unter aeroben Bedingungen in flüssigem Nährmedium züchtet.

4. Verfahren gemäss Anspruch 3, dadurch gekennzeichnet, dass man den Rekombinantenstamm Nocardia mediterranei DMS 1415 unter aeroben Bedingungen in flüssigem Nährmedium züchtet.

5. Verfahren gemäss einem der Ansprüche 1–4, dadurch gekennzeichnet, dass man in einem wässrigen Nährmedium enthaltend eine Kohlenstoffquelle, eine Stickstoffquelle und Mineralsalze züchtet.

6. Verfahren gemäss einem der Ansprüche 1–5, dadurch gekennzeichnet, dass man 3-Hydroxyrifamycin-S produziert.

7. Verfahren gemäss einem der Ansprüche 1–5, dadurch gekennzeichnet, dass man 3,31-Dihydroxyrifamycin-S produziert.

8. Verfahren gemäss einem der Ansprüche 1–5, dadurch gekennzeichnet, dass man 1-Desoxy-1-oxarifamycin-S produziert.

9. Verfahren zur Herstellung von pharmazeutischen Präparaten enthaltend mindestens eine der in den Ansprüchen 6–8 angegebenen Verbindungen.

10. Verfahren gemäss Anspruch 9 zur Herstellung von pharmazeutischen Präparaten enthaltend 3-Hydroxyrifamycin-S.

**Claims for the contracting states: BE, CH, DE, FR, GB, IT, LU, NL, SE**

1. A process for the preparation of 3-hydroxyrifamycin S, 3,31-dihydroxyrifamycin S and 1-desoxy-1-oxarifamycin S, characterised by cultivating, under aerobic conditions in a liquid nutrient medium, a strain of Nocardia mediterranei, which is derived from Streptomyces mediterranei ATCC 13 685 as the parent strain and is characterised by the ability to produce at least one of the above-mentioned compounds, and by the isolation of at least one of the above-mentioned final substances from the resultant fermentation material.

2. A process for the preparation of a fermentation material containing 3-hydroxyrifamycin S, 3,31-dihydroxyrifamycin S and/or 1-desoxy-1-oxyrifamycin S, characterised by cultivating, under aerobic conditions in a liquid nutrient medium, a strain of Nocardia mediterranei which is derived from Streptomyces mediterranei ATCC 13 685 as the parent strain, and characterised by the ability to produce at least one of the mentioned compounds.

3. A process according to either of claims 1 or 2, characterised in that a recombinant strain of Nocardia mediterranei which is derived from Streptomyces mediterranei ATCC 13 685 as the parent strain, is resistant to streptomyces and autotrophic with respect to amino acids and produces at least one of the mentioned compounds, is cultivated under aerobic conditions in a liquid nutrient medium.

4. A process according to claim 3, characterised in that the recombinant strain Nocardia mediterranei DMS 1415 is cultivated under aerobic conditions in a liquid nutrient medium.

5. A process according to any one of claims 1 to 4, characterised in that the cultivation is effected in an aqueous nutrient medium containing a carbon source, a nitrogen source and mineral salts.

6. The recombinant strain Nocardia mediterranei DMS 1514.

7. 3-Hydroxyrifamycin S.

8. 3,31-Dihydroxyrifamycin S.

9. 1-Desoxy-1-oxarifamycin S.

10. The use of 3-hydroxyrifamycin S, 3,31-dihydroxyrifamycin S and/or 1-desoxy-1-oxarifamycin S as an antibiotic.

11. Use of 3-hydroxyrifamycin S, 3,31-dihydroxyrifamycin S and/or 1-desoxy-oxarifamycin S singly or together in any combination, for combating infectious bacterial diseases outside the human and animal body.

12. A pharmaceutical preparation containing at least one of the compounds claimed in any one of claims 7 to 9.

**Claims for the contracting state: AT**

1. A process for the preparation of 3-hydroxyrifamycin S, 3,31-dihydroxyrifamycin S and 1-desoxy-1-oxarifamycin S, characterised by cultivating, under aerobic conditions in a liquid nutrient medium, a strain of Nocardia mediterranei, which is derived from Streptomyces mediterranei ATCC 13 685 as the parent strain and is characterised by the ability to produce at least one of the abovementioned compounds, and by the isolation of at least one of the above-mentioned final substances from the resultant fermentation material.

2. A process for the preparation of a fermentation material containing 3-hydroxyrifamycin S, 3,31-dihydroxyrifamycin S and/or 1-desoxy-1-oxarifamycin S, characterised by cultivating, under aerobic conditions in a liquid nutrient medium, a strain of Nocardia mediterranei which is derived from Streptomyces mediterranei ATCC 13 685 as the parent strain, and characterised by the ability to produce at least one of the mentioned compounds.

3. A process according to either of claims 1 or 2, characterised in that a recombinant strain of Nocardia mediterranei which is derived from Streptomyces mediterranei ATCC 13 685 as the parent strain, is resistant to streptomycin and autotrophic with respect to amino acids and produces at least one of the mentioned compounds, is cultivated under aerobic conditions in a liquid nutrient medium.

4. A process according to claim 3, characterised in that the recombinant strain Nocardia mediterranei DMS 1415 is cultivated under aerobic conditions in a liquid nutrient medium.

5. A process according to any one of claims 1 to 4, characterised in that the cultivation is effected in an aqueous nutrient medium containing a carbon source, a nitrogen source and mineral salts.

6. A process according to any one of claims 1 to 5 for the preparation of 3-hydroxyrifamycin S.

7. A process according to any one of claims 1 to 5 for the preparation of 3,31-dihydroxyrifamycin S.

8. A process according to any one of claims 1 to 5 for the preparation of 1-desoxy-1-oxarifamycin S.

9. A process for the production of a pharmaceutical preparation containing at least one of the compounds claimed in any one of claims 6 to 8.

10. A process according to claim 9 for the production of a pharmaceutical preparation containing 3-hydroxyrifamycin S.

**Revendications pour les Etats contractants: BE, CH, DE, FR, GB, IT, LU, NL, SE**

1. Procédé de préparation de 3-hydroxyrifamycine-S, de 3,31-dihydroxyrifamycin-S et de 1-désoxy-1-oxarifamycin-S, caractérisé en ce qu'on cultive une souche de Nocardia mediterranei qui dérive de Streptomyces mediterranei ATCC 13 685 comme souche de base et qui est caractérisée par l'aptitude à produire au moins l'un des composés mentionnés ci-dessus, dans des conditions aérobies dans un milieu nutritif liquide, et en ce qu'on isole au moins l'un des produits finals mentionnés ci-dessus à partir de la matière de fermentation obtenue.

2. Procédé de préparation d'une matière de fermentation contenant de la 3-hydroxyrifamycine-S, de la 3,31-dihydroxyrifamycine-S et/ou de la 1-désoxy-1-oxarifamycine-S, caractérisé en ce qu'on cultive une souche de Nocardia mediterranei, qui est dérivée de Streptomyces mediterranei ATCC 13 685 comme souche de base, et qui est caractérisée par l'aptitude à produire au moins l'un des composés mentionnés, dans des conditions aérobies dans un milieu nutritif liquide.

3. Procédé selon l'une des revendications 1 ou 2, caractérisé en ce qu'on cultive une souche de recombinants produisant au moins l'un des composés mentionnés, autotrophe vis-à-vis des acides aminés et résistant à la streptomycine, de Nocardia mediterranei, dérivée de Streptomyces mediterranei ATCC 13 685 comme souche de base, dans des conditions aérobie dans un milieu nutritif liquide.

4. Procédé selon la revendication 3, caractérisé en ce qu'on cultive la souche de recombinante Nocardia mediterranei DMS 1415 dans des conditions aérobies en milieu nutritif liquide.

5. Procédé selon l'une des revendications 1 à 4, caractérisé en ce qu'on cultive dans un milieu nutritif aqueux contenant une source de carbone, une source d'azote et des sels minéraux.

6. Souche de recombinants Nocardia mediterranei DMS 1415.

7. 3-hydroxyrifamycine-S.

8. 3,31-dihydroxyrifamycine-S.

9. 1-désoxy-1-oxarifamycine-S.

10. 3-hydroxyrifamycine-S, 3,31-dihydroxyrifamycine-S et/ou 1-désoxy-1-oxarifamycine-S aux fins d'application comme antibiotique.

11. Application de 3-hydroxyrifamycine-S, de 3,31-dihydroxyrifamycine-S et/ou de 1-désoxy-1-oxarifamycine-S, isolément ou ensemble dans une combinaison quelconque pour lutter contre les infections bactériennes à l'extérieure du corps humain et animal.

12. Préparations pharmaceutiques contenant au moins un des composés mentionnés dans les revendications 7 à 9.

**Revendications pour l'Etat contractant: AT**

1. Procédé de préparation de 3-hydroxyrifamycine-S, de 3,31-dihydroxyrifamycine-S et de 1-désoxy-1-oxarifamycine-S, caractérisé en ce qu'on cultive une souche de Nocardia mediterranei qui dérive de Streptomyces mediterranei ATCC 13 685 comme souche de base et par l'aptitude à produire au moins l'un des composés mentionnés ci-dessus, dans des conditions aérobies dans un milieu nutritif liquide et en ce qu'on isole au moins l'un des produits finals mentionnés ci-dessus à partir de la matière de fermentation obtenue.

2. Procédé de préparation d'une matière de fermentation contenant de la 3-hydroxyrifamycine-S, de la 3,31-dihydroxyrifamycine-S et/ou de la 1-désoxy-1-oxarifamycine-S, caractérisé en ce qu'on cultive une souche de Nocardia mediterranei, qui dérive de Streptomyces mediterranei ATCC 13 685 comme souche de base, et qui est

caractérisée par l'aptitude à produire au moins l'un des composés mentionnés, dans des conditions aérobies dans un milieu nutritif liquide.

3. Composé selon l'une des revendications 1 ou 2, caractérisé en ce qu'on cultive une souche de recombinants, productrice d'au moins un des composés mentionnés, autotrophe par rapport aux acides aminés et résistante à la streptomycine, de Nocardia mediterranei, dérivée de Streptomyces mediterranei ATCC 13 685 comme souche de base, dans des conditions aérobies en milieu nutritif liquide.

4. Procédé selon la revendication 3, caractérisé en ce qu'on cultive la souche de recombinants Nocardia mediterranei DMS 1415 dans des conditions aérobies en milieu nutritif liquide.

5. Procédé selon l'une des revendications 1–4, caractérisé en ce qu'on cultive dans un milieu nutritif aqueux contenant une source de carbone, une source d'azote et des acides minéraux.

6. Procédé selon l'une des revendications 1–5, caractérisé en ce qu'on produit la 3-hydroxyrifamycine-S.

7. Procédé selon l'une des revendications 1–5, caractérisé en ce qu'on produit la 3,31-dihydroxyrifamycine-S.

8. Procédé selon l'une des revendications 1–5, caractérisé en ce qu'on produit la 1-désoxy-1-oxarifamycine-S.

9. Procédé de préparation de préparations pharmaceutiques contenant au moins l'un des composés mentionnés dans les revendications 6–8.

10. Procédé selon la revendication 9 de préparation de préparations pharmaceutiques contenant la 3-hydroxyrifamycine-S.

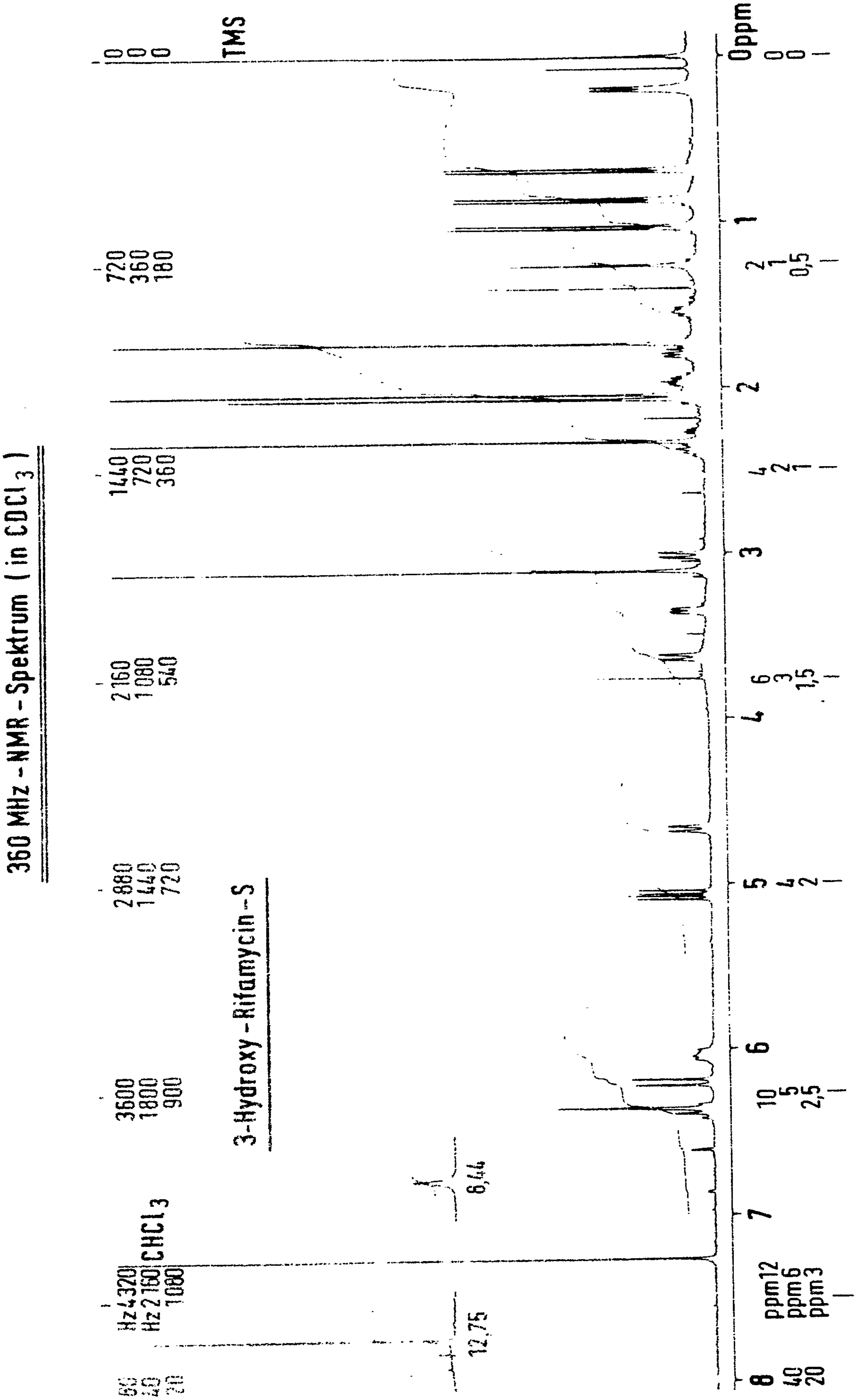
360 MHz - NMR - Spektrum ( in CDCl3 )
3-Hydroxy-Rifamycin-S
TMS
CHCl3